(19) Europäisches Patentamt
European Patent Office
Office européen des brevets

(11) Publication number: **0 336 561 B1**

(12) # EUROPEAN PATENT SPECIFICATION

(45) Date of publication of patent specification: **28.07.93**

(51) Int. Cl.5: **C07D 213/59**, A61K 31/44, C07D 213/55

(21) Application number: **89302143.6**

(22) Date of filing: **03.03.89**

(54) **2-Pyridylacetic acid derivatives, preparations thereof, and compositions containing them.**

(30) Priority: **03.03.88 US 163711**

(43) Date of publication of application:
**11.10.89 Bulletin 89/41**

(45) Publication of the grant of the patent:
**28.07.93 Bulletin 93/30**

(84) Designated Contracting States:
**DE FR GB IT**

(56) References cited:
**EP-A- 0 223 512**

(73) Proprietor: **SUNTORY LIMITED**
**1-40, Dojimahama 2-chome**
**Kita-ku, Osaka-shi, Osaka-fu 530(JP)**

(72) Inventor: **Okitsu, Mitsuhito**
**5-18-404, Wakayamadai 1-chome Shimamoto-cho**
**Mishima-gun Osaka(JP)**

(74) Representative: **Harrison, David Christopher et al**
**MEWBURN ELLIS 2 Cursitor Street**
**London EC4A 1BO (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 336 561 B1

## Description

The present invention relates to a 2-pyridylacetic acid derivative having the formula (I):

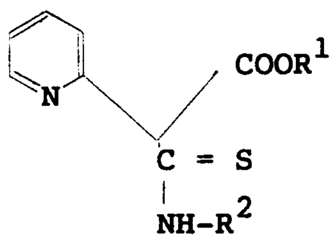

(I)

wherein $R^1$ represents $-(CH_2)_m$-cycloalkyl group having $C_5$ - $C_8$ cycloalkyl, which may be substituted with at least one alkyl group having 1 to 6 carbon atoms, where m represents zero or an integer of 1 to 4; $R^2$ represents hydrogen, or a linear alkyl having 1 to 10 carbon atoms. In other aspects it provides processes for preparation thereof, and pharmaceutical compositions or agents containing them. More specifically, such 2-pyridylacetic acid derivatives and their pharmacologically acceptable acid addition salts are novel compounds useful as therapeutical agents for peptic ulcers, since they have the effect of inhibiting attacking factors and the effect of potentiating defending factors of peptic ulcer and also have a low toxicity.

The etiology of peptic ulcer has been discussed in terms of an imbalance between aggressive and defensive factors, but the factors which increase the resistance of tissue have not yet been clarified. Accordingly, the maxim "no acid, no ulcer" still remains true, and under the present situation, the therapy target of peptic ulcers is still directed to a control of gastric acid.

In recent years, potent inhibitors of gastric acid secretion such as histamine $H_2$ receptor antagonist (cimetidine, ranitidine, famotidine) and anticholinergics of gastric acid (pirenzepine) have been introduced to therapeutics for gastric and duodenal ulcer patients. However, these are not sufficient for preventing a worsening or recurrence of an ulcer.

As mentioned above, a satisfactory effect cannot be obtained in the therapy of an ulcer merely by the use of a drug which can prevent the generation of an ulcer, i.e., inhibit aggressive factors. Accordingly, under the present situation, a drug inhibiting aggressive factors and a protective drug for gastric mucosa are respectively selected or used in combinations of both types as the ulcer therapeutical agent, depending on the conditions of the disease. Although some compounds stated to have both such effects have been proposed, in practice these have proved to have a weak inhibiting acid secretion, and primarily to have a protective effect for gastric mucosa.

As described above, the development of a potent anti-peptic ulcer drug well balanced in both the actions of inhibition of aggressive factors and protection of gastric mucosa is strongly desired. Furthermore, it is also desired that such a drug should have a very low toxicity and a minimum of side effects as a drug for peptic ulcer disease.

In the prior art, our own EP-A-223512 describes, for treatment of peptic ulcer, a 2-pyridylacetic acid derivative of the formula:

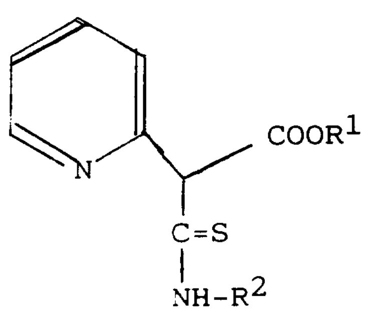

(I)

in which R' is $C_{1-15}$ alkyl, $C_{5-20}$ alkenyl or $C_{7-15}$ aralkyl; $R^2$ is hydrogen, a linear or cyclic alkyl, a hydroxyalkyl group, an alkenyl, an aryl, an aralkyl or a group $-(CH_2)_n$-A, where n is 0 to 3 and A is a nitrogen-containing heterocyclic group which may be substituted with $C_{1-10}$ alkyl or $C_{7-10}$ aralkyl.

The problem addressed by the present invention is to provide novel compounds with pharmaceutical applicability in particular for the treatment of peptic ulcer, and desirably having the low toxicity and well balanced activities referred to above.

In one aspect of the invention, there are provided 2-pyridylacetic acid derivatives having the above-mentioned formula (I) and pharmacologically acceptable acid addition salts thereof.

In another aspect of the invention, there is provided a process for preparing a compound having the formula (I), or a pharmacologically acceptable acid addition salt thereof, which comprises reacting an addition product having the formula (III):

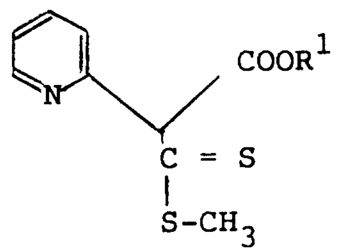

(III)

wherein $R^1$ is as defined above; with ammonia or an amine represented by the formula (IV):

$R^2 - NH_2$ (IV)

wherein $R^2$ is as defined above, followed by treatment with a pharmacologically acceptable acid, if desired.

In a further aspect of the invention, there is provided a peptic ulcer therapeutical agent comprising the 2-pyridylacetic acid derivative having the formula (I) and/or a pharmacologically acceptable acid addition salt thereof as the active ingredient; also use of such a compound in the manufacture of such a medicament.

The novel compound 2-pyridylacetic acid derivative having the above-mentioned formula (I) and its pharmacologically acceptable acid addition salt according to the present invention has a protecting action for gastric mucosa together with an effect of inhibiting gastric acid secretion, and has a low toxicity, and therefore, is a useful substance which can be used for the therapy of a peptic ulcer.

A compound having the above-mentioned formula (I) of the present invention may be prepared as follows:

That is, a 2-pyridylacetic acid ester having the formula (II);

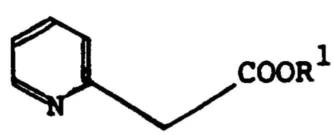

(II)

wherein $R^1$ is as defined above, is allowed to react with carbon disulfide in an organic solvent in the presence of a base at a temperature of -78 °C to 0 °C. The reaction is completed within several minutes to several tens of minutes. After completion of the reaction, methyl iodide is added and stirring is continued for several hours, whereby an addition product having the formula (III):

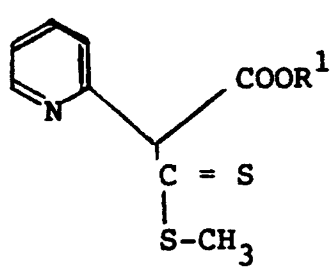

(III)

wherein $R^1$ is as defined above, can be obtained.

The solvent usable in the above reaction, may include, for example, an ether such as tetrahydrofuran, diethyl ether, dimethoxyethane or dioxane, or an aromatic hydrocarbon such as benzene, toluene or xylene, or dimethyl sulfoxide. Alternatively, the base usable in the above reaction may preferably include an alkyl lithium reagent, sodium amide, sodium hydride, potassium hydride, potassium t-butoxide, a sodium alcoholate, a potassium alcoholate, metallic sodium, and the like.

The amount of the base to be used in the above-mentioned reaction is not particularly limited, but may be, for example, 1 to 1.2 equivalent relative to the above compound (II).

The thus-obtained addition product having the formula (III) can be purified by any conventional purification method, for example, chromatography, recrystallization or distillation.

When the above compound (III) is allowed to react with ammonia or an amine having the formula (IV):

$R^2 - NH_2$ (IV)

wherein $R^2$ is as defined above, in water and an organic solvent, or in an organic solvent, for 1 to 10 hours, the present compound can be obtained. The solvent usable in this reaction is not particularly limited, unless the reaction is adversely affected, but preferably, for example, water, an alcoholic solvent, a chlorine type solvent, an aromatic hydrocarbon solvent, an ether solvent, or an acetic acid ester solvent is used.

After completion of the reaction, the desired compound can be purified by, for example, recrystallization, column chromatography, or alternatively it can be treated with a pharmacologically acceptable acid and converted into an acid addition salt, which, in turn, can be purified by recrystallization or chromatography.

The acids usable for the preparation of the acid addition salts of the above 2-pyridylacetic acid derivative according to the present invention may include, for example, inorganic acids such as hydrochloric acid, hydrobromic acid, sulfuric acid, phosphoric acid, perchloric acid, and the like, and organic acids such as acetic acid, oxalic acid, citric acid, lactic acid, maleic acid, succinic acid, fumaric acid, tartaric acid, gluconic acid, mandelic acid, methanesulfonic acid, and the like.

Another process for preparing the 2-pyridylacetic acid derivative according to the present invention comprises dissolving a compound having the above formula (II) in an organic solvent and treating it with a base at a temperature of 0 °C or lower. Examples of such organic solvents are, preferably, ether solvents and aromatic hydrocarbon solvents. The amount of the base used in the above reaction is not particularly limited, but is preferably used at 1.0 to 1.2 equivalent relative to the compound of the above formula (II). Examples of such bases are, preferably, sodium hydride, a sodium alkoxide, a potassium alkoxide, sodium amide, n-butyllithium, and metallic sodium.

In the next step, an isothiocyanate having the formula (V):

$S = C = N - R^3$ (V)

wherein $R^3$ represents an alkyl group, is added to the abovementioned reaction mixture, whereby, a compound according to the present invention having the formula (Ia):

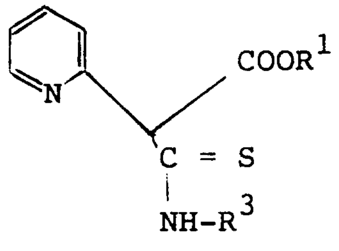

(Ia)

wherein $R^1$ and $R^3$ are as defined above, can be obtained.

The novel 2-pyridylacetic acid having the above formula (I) according to the present invention may be administered as it is, or may be formed in various kinds of dosage forms by utilizing known preparation methods. For example, for oral administration, it can be generally formed into preparations such as tablets, powders, granules, capsules, syrup, and the like, or for parenteral administration, can be injected or filled in suppositories, and the like. In either case, preparations with various forms can be obtained by mixing with known liquid or solid excipients or carriers conventionally used in the preparation.

Examples of such excipients or carriers include polyvinyl pyrrolidone, gum arabic, gelatin, sorbitol, cyclodextrins,tragacanth, magnesium stearate, talc, polyethylene glycol, polyvinyl alcohol, silica, lactose, crystalline cellulose, sugar, starch, calcium phosphate, vegetable oil, carboxymethyl cellulose, sodium lauryl sulfate, water, ethanol, glycerine, mannitol, syrup, and the like.

A peptic ulcer therapeutical agent of the present invention should contain the compound having the formula (I), or a pharmacologically acceptable acid addition salt thereof, in an effective amount.

An effective amount of the peptic ulcer therapeutical agent of the present invention to be administered may vary depending on the condition and the age of the patient to be treated, the administration route, the dosage form, the number of administrations, and the like, but may be, for example, generally within the scope of from about 5 to 1,000 mg, preferably from 10 to 500 mg, for a human adult per day.

## EXAMPLES

The present invention will now be further illustrated by the following Examples describing embodiments thereof.

Compounds having the above formula (I) of the present invention were synthesized according to the two methods as described below. The method incorporating the formula (III) is called method B, and the method for obtaining the compound (Ia) of the present invention by allowing an isothiocyanate to react directly with the formula (II) is called method A.

### Example 1

### Synthesis of cyclopentylmethyl 2-pyridylacetate

A mixture of 2-pyridylacetic acid hydrochloride 6.06 g (34.94 mmol), cyclopentylmethanol 3.50 g (34.94 mmol) and 1-ethyl-3-(3-dimethylaminopropyl)-carbodiimide hydrochloride 10.03 g (52.35 mmol) in 12 ml of pyridine and 50 ml of methylene chloride was stirred at room temperature for 2 hours.

The reaction mixture was quenched with 200 ml of water, and extracted with chloroform. The extract was washed with water and dried over anhydrous magnesium sulfate. The residue obtained by evaporation of the solvent was distilled to give 5.50 g of the desired compound as an yellow oil.

The various compounds listed in Table 1 were prepared in the same manner as in Example 1. The physical properties of these starting compounds are also summarized in Table 1.

Table 1

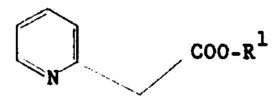

| No. | $R^1$ | b.p. | IR $\nu$ $cm^{-1}$ | NMR($CDCl_3$)$\sigma$: | Yield % |
|---|---|---|---|---|---|
| 1 | 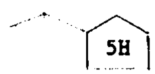 | yellow oil b.p.$_{1.2}$ 120°C | 2950, 2860, 1730, 1590, 1470, 1430, 1250, 1155, 990, 745 (NaCl) | 1.05 - 1.80 (8H, m), 2.19 (1H, m), 3.85 (2H, s), 4.01 (2H, d, J = 6.6 Hz), 7.19 (1H, m), 7.30 (1H, d, J = 7.9 Hz), 7.65 (1H, m), 8.56 (1H, d, J = 4.6 Hz) | 72 |
| 2 | 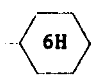 | yellow oil b.p.$_{1.2}$ 117 - 119°C | 2940, 2850, 1730, 1590, 1430, 1250, 1160, 1005, 965, 750 (NaCl) | 1.17 - 1.85 (10H, m), 3.85 (2H, s), 4.81 (1H, m), 7.20 (1H, m), 7.32 (1H, d, J = 7.9 Hz), 7.67 (1H, m), 8.56 (1H, d, J = 4.6 Hz) | 74 |
| 3 | 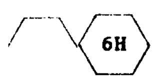 | yellow oil b.p.$_1$ 140 - 142°C | 2920, 2850, 1730, 1590, 1430, 1250, 1150, 990, 740 (NaCl) | 0.82 - 1.75 (11H, m), 3.86 (2H, s), 3.93 (2H, 6.6 Hz), 7.20 (1H, m), 7.30 (1H, d, J = 7.9 Hz), 7.66 (1H, m), 8.56 (1H, d, J = 4.6 Hz) | 85 |

Table 1 (Continued)

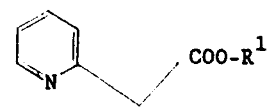

| No. | $R^1$ | b.p. | IR $\nu$ $cm^{-1}$ | NMR($CDCl_3$)σ: | Yield % |
|---|---|---|---|---|---|
| 4 | 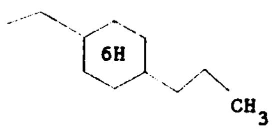 | yellow oil b.p.$_{1.5}$ 163°C | 2920, 2840, 1730, 1590, 1430, 1330, 1250, 1150, 990, 740 (NaCl) | 0.76 - 1.76 (17H, m), 3.88 (2H, s), 3.94 (2H, d, J = 6.6 Hz), 7.21 (1H, m), 7.32 (1H, d, J = 7.9 Hz), 7.69 (1H, m), 8.57 (1H, d, J = 5.9 Hz) | 75 |
| 5 | 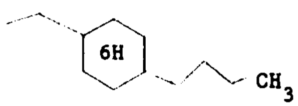 | yellow oil b.p.$_{1.5}$ 169°C | 2900, 2840 1730, 1580, 1330, 1250, 1150, 990, 740 (NaCl) | 0.79 - 1.80 (19H, m), 3.88 (2H, s), 3.94 (2H, d, J = 6.6 Hz), 7.21 (1H, m), 7.33 (1H, d, J = 7.9 Hz), 7.69 (1H, m), 8.56 (1H, d, J = 5.9 Hz) | 74 |
| 6 | 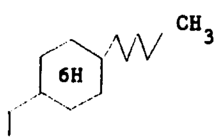 | yellow oil b.p.$_{1.5}$ 179°C | 2930, 2850, 1740, 1590, 1440, 1340, 1250, 1150, 740 (NaCl) | 0.76 - 1.82 (21H, m), 3.89 (2H, s), 3.94 (2H, d, J = 5.9 Hz), 7.23 (1H, m), 7.33 (1H, d, J = 7.9 Hz), 7.70 (1H, m), 8.57 (1H, d, J = 4.6 Hz) | 74 |

Table 1 (Continued)

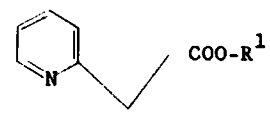

| No. | $R^1$ | b.p. | IR $\nu$ $cm^{-1}$ | NMR($CDCl_3$)σ: | Yield % |
|---|---|---|---|---|---|
| 7 | 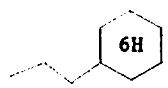 | yellow oil b.p.$_{1.3}$ 155 - 157°C | 2920, 2840, 1730, 1580, 1250, 1150, 990, 740 (NaCl) | 0.82 - 1.72 (13H, m), 3.85 (2H, s), 4.16 (2H, t, J = 7.3 Hz), 7.17 - 7.31 (2H, m), 7.67 (1H, m), 8.56 (1H, d, J = 3.9 Hz) | 74 |
| 8 | 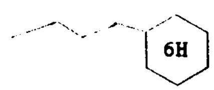 | yellow oil b.p.$_{0.5}$ 151°C | 2920, 2850, 1740, 1590, 1440, 1335, 1250, 1150, 745 (NaCl) | 0.82 - 1.75 (15H, m), 3.84 (2H, s), 4.09 (2H, t, J = 7.3 Hz), 7.18 (1H, m), 7.29 (1H, d, J = 7.9 Hz), 7.65 (1H, m), 8.56 (1H, d, J = 4.0 Hz) | 84 |
| 9 | 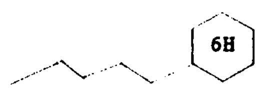 | yellow oil b.p.$_{1.5}$ 165°C | 2920, 2850, 1740, 1590, 1450, 1335, 1250, 1050, 740 (NaCl) | 0.75 - 1.70 (17H, m), 3.84 (2H, s), 4.11 (2H, t, J = 6.6 Hz), 7.18 (1H, m), 7.29 (1H, d, J = 7.9 Hz), 7.65 (1H, t, d, J = 7.9 Hz, J = 2.0 Hz), 8.55 (1H, d, J = 4.0 Hz) | 81 |

Table 1 (Continued)

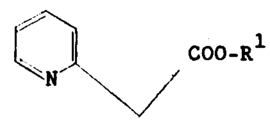

| No. | $R^1$ | b.p. | IR $\nu$ $cm^{-1}$ | NMR($CDCl_3$)$\sigma$: | Yield % |
|---|---|---|---|---|---|
| 10 | 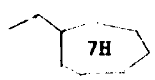 | yellow oil b.p.$_{1.2}$ 144 - 148°C | 2920, 2860, 1740, 1595, 1470, 1440, 1260, 1160, 1000, 750 (NaCl) | 1.10 - 1.87 (13H, m), 3.85 (2H, s), 3.92 (2H, d, J = 6.6 Hz), 7.19 (1H, m), 7.30 (1H, d, J = 7.9 Hz), 7.65 (1H, m), 8.56 (1H, d, J = 4.6 Hz) | 78 |

Example 2-A

Synthesis of cyclopentylmethyl 2-methylthiocarbamoyl-2-(2-pyridyl)acetate

(Synthesis according to method A)

A 2.0 g (9.6 mmol) amount of iso-amyl 2-pyridylacetate was dissolved in 40 ml of dry tetrahydrofuran, and to the resultant solution were added, under a nitrogen gas stream and at a temperature of -78°C to 0°C, 1.1 equivalent of n-butyl lithium solution in hexane or 1.1 equivalent of powdery sodium amide, followed by stirring for 15 to 30 minutes.

To the resultant solution was added 0.44 g (60.19 mmol) of methylisothiocyanate, and the mixture was stirred at room temperature for 2 hours. Water was then added to the reaction mixture, and the mixture was extracted with chloroform. The organic layer was then washed with water, and dried over anhydrous magnesium sulfate.

The residue obtained by evaporation of the solvent was subjected to column chromatography on silica gel to obtain an yellow oil, which was recrystallized from chloroform: n-hexane to give 0.82 g of colorless prisms.

Example 2-B

Synthesis of cyclopentylmethyl 2-(2-pyridyl)-2-thiocarbamoyl acetate hydrochloride

(Synthesis according to method B)

A 4.00 g (18.24 mmol) amount of cyclopentylmethyl 2-pyridylacetate was dissolved in 50 ml of dry tetrahydrofuran, and to the resultant solution was added, under a nitrogen stream at -78°C, 1.1 equivalent of n-butyl lithium solution in hexane. After 15 minutes, 1.52 g (20.06 mmol) of carbon disulfide was added, and further, after stirring for 15 minutes, 2.84 g (20.06 mmol) of methyl iodide was added, followed by stirring for 2 hours.

Water was added to the reaction mixture obtained above, and the mixture was extracted with chloroform. The organic layer was then washed with water, followed by drying over anhydrous magnesium sulfate.

The residue obtained by evaporation of the solvent was subjected to column chromatography on silica gel to obtain cyclopentylmethyl 2-dithiomethoxycarbonyl-2-(2-pyridyl) acetate as a yellow oil (yield 81%).

The resultant product was dissolved in 30 ml of dioxane, excess ammonia gas was bubbled into the solution for 20 min. at room temperature, and the mixture was further stirred for 5 hours. The residue obtained by concentration was subjected to column chromatography on silica gel to obtain an oil, which was converted to 2.64 g of the HCl salt (yield 46%).

The various compounds listed in Table 2 were prepared in the same manner as in Example 2. The physical properties of these compounds are summarized in Table 2.

The compounds thus obtained are as follows:

1. Cyclopentylmethyl 2-(2-pyridyl)-2-thiocarbamoylacetate hydrochloride
2. Cyclopentylmethyl 2-methylthiocarbamoyl-2-(2-pyridyl)acetate
3. Cyclohexyl 2-(2-pyridyl)-2-thiocarbamoylacetate hydrochloride
4. Cyclohexyl 2-methylthiocarbamoyl-2-(2-pyridyl)acetate
5. Cyclohexylmethyl 2-(2-pyridyl)-2-thicarbamoylacetate hydrochloride
6. Cyclohexylmethyl 2-methylthiocarbamoyl-2-(2-pyridyl)acetate hydrochloride
7. (trans-4-n-Propylcyclohexyl)methyl 2-(2-pyridyl)-2-thiocarbamoylacetate hydrochloride
8. (trans-4-n-Propylcyclohexyl)methyl 2-methyl-thiocarbamoyl-2-(2-pyridyl)acetate hydrochloride
9. (trans-4-n-Butylcyclohexyl)methyl 2-(2-pyridyl)-2-thiocarbamoylacetate hydrochloride
10. (trans-4-n-Butylcyclohexyl)methyl 2-methylthiocarbamoyl-2-(2-pyridyl)acetate hydrochloride
11. (trans-4-n-Pentylcyclohexyl)methyl 2-(2-pyridyl)-2-thiocarbamoylacetate hydrochloride
12. (trans-4-n-Pentylcyclohexyl)methyl 2-methylthiocarbamoyl-2-(2-pyridyl)acetate hydrochloride
13. Cyclohexylethyl 2-(2-pyridyl)thiocarbamoylacetate hydrochloride
14. Cyclohexylethyl 2-methylthiocarbamoyl-2-(2-pyridyl)acetate hydrochloride
15. Cyclohexylpropyl 2-(2-pyridyl)-2-thiocarbamoylacetate hydrochloride
16. Cyclohexylpropyl 2-methylthiocarbamoyl-2-(2-pyridyl)acetate hydrochloride
17. Cyclohexylbutyl 2-(2-pyridyl)-2-thiocarbamoylacetate hydrochloride

18. Cyclohexylbutyl 2-methylthiocarbamoyl-2-(2-pyridyl)acetate hydrochloride
19. Cycloheptylmethyl 2-(2-pyridyl)-2-thiocarbamoylacetate hydrochloride
20. Cycloheptylmethyl 2-methylthiocarbamoyl-2-(2-pyridyl)acetate

Table 2

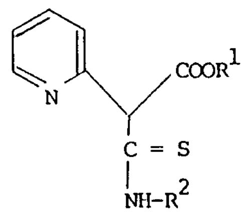

| No. | $R^1$ | $R^2$ | mp°C | IR ν $cm^{-1}$ | NMR($CDCl_3$)σ: | method & yield (%) |
|---|---|---|---|---|---|---|
| 1 | 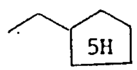 | H | colorless prisms HCl salt 114 - 116 | 3300, 2950, 1750, 1630, 1450, 1300, 1180, 750 (KBr) | 1.21 - 1.86 (8$^{11}$, m), 2.27 (1H, m), 4.12 (2H, m), 6.63 (1H, s), 7.59 (1H, br.s), 7.89 (1H, m), 8.45 (1H, m), 8.56 (1H, d, J = 5.3 Hz), 8.73 (1H, d, J = 7.9 Hz), 10.00 (1H, br.s) | B 46 |
| 2 | 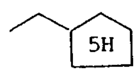 | $CH_3$ | colorless prisms 62 - 64 | 3150, 2950, 1750, 1570, 1480, 1380, 1160, 750 (KBr) | 1.08 - 1.17 (2H, m), 1.45 - 1.67 (6H, m), 2.13 (1H, m), 3.23 (3H, d, J = 5.3 Hz), 4.00 (2H, m), 5.41 (1H, s), 7.29 (1H, d.d, J = 4.6 Hz, 1.3 Hz), 7.44 (1H, d, J = 7.3 Hz), 7.73 (1H, t.d, J = 7.3 Hz, J = 1.3 Hz), 8.56 (1H, d, J = 4.6 Hz), 10.24 (1H, br.s) | A 51 |
| 3 | 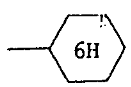 | H | HCl salt colorless prisms 134 - 145 | 3270, 2930, 2600, 1750, 1630, 1445, 1290, 1180, 1005, 750 (KBr) | 1.20 - 2.00 (10H, m), 4.91 (1H, m), 6.58 (1H, s), 7.64 (1H, br.s), 7.89 (1H, m), 8.45 (1H, m), 8.59 (1H, d, J = 5.9 Hz), 8.75 (1H, d, J = 7.9 Hz), 9.98 (1H, br.s) | B 37 |

Table 2 (Continued)

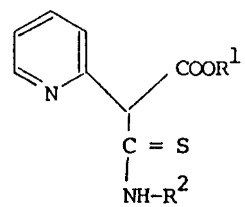

| No. | $R^1$ | $R^2$ | mp°C | IR ν $cm^{-1}$ | NMR($CDCl_3$)σ: | method & yield (%) |
|---|---|---|---|---|---|---|
| 4 | 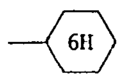 | $CH_3$ | colorless prisms 85 - 86 | 3170, 2930, 1750, 1565, 1475, 1290, 1170, 750 (KBr) | 1.16 - 1.86 (10H, m), 3.22 (3H, d, J = 5.3 Hz), 4.81 (1H, m), 5.47 (1H, s), 7.31 (1H, m), 7.55 (1H, d, J = 7.9 Hz), 7.77 (1H, m), 8.55 (1H, d, J = 4.6 Hz), 10.26 (1H, br.s) | A 33 |
| 5 | 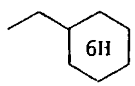 | H | colorless prisms HCl salt 135 - 144 | 3050, 2930, 1730, 1625, 1460, 1440, 1180, 1005, 770, 750 (KBr) | 0.90 - 1.33 (5H, m), 1.66 - 1.84 (6H, m), 3.94 - 4.13 (2H, m), 6.61 (1H, s), 7.80 (1H, br.s), 7.89 (1H, br.t, J = 6.6 Hz), 8.44 (1H, br.t, J = 7.9 Hz), 8.63 (1H, d, J = 5.3 Hz), 8.73 (1H, d, J = 8.8 Hz), 10.0 (1H, br.s) | B 64 |
| 6 | 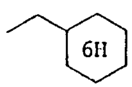 | $CH_3$ | colorless prisms HCl salt 131 - 139 | 3400, 3160, 2920, 1730, 1610, 1530, 1480, 1390, 1170, 1080, 750 (KBr) | 0.89 - 1.81 (11H, m), 3.13 (3H, d, J = 4.6 Hz), 4.05 (2H, m), 6.63 (1H, s), 7.86 (1H, m), 8.41 (1H, m), 8.52 (1H, m), 8.78 (1H, d, J = 8.6 Hz), 10.50 (1H, br.s) | A 72 |

Table 2 (Continued)

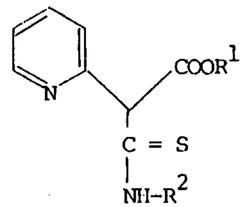

| No. | $R^1$ | $R^2$ | mp°C | IR ν $cm^{-1}$ | NMR($CDCl_3$)σ: | method & yield (%) |
|---|---|---|---|---|---|---|
| 7 | 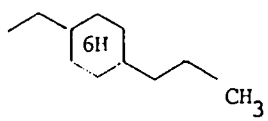 | H | HCl salt colorless prisms 138 - 144 | 3360, 2920, 2600, 1740, 1630, 1545, 1450, 1300, 1190, 950, 760 (KBr) | 0.82 - 1.87 (17H, m), 4.04 (2H, m), 6.65 (1H, s), 7.62 (1H, br.s), 7.90 (1H, m), 8.45 (1H, t.d, J = 7.9 Hz, J = 1.3 Hz), 8.57 (1H, d, J = 5.9 Hz), 8.74 (1H, d, J = 7.9 Hz), 10.01 (1H, br.s) | B 53 |
| 8 | 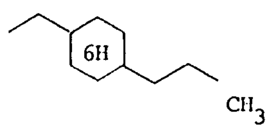 | $CH_3$ | HCl salt colorless prisms 117 - 122 | 3150, 2920, 2550, 1550, 1460, 1380, 1290, 1210, 1170, 740 (KBr) | 0.77 - 1.83 (17H, m), 3.13 (3H, d, J = 5.3 Hz), 4.03 (2H, m), 6.64 (1H, s), 7.82 (1H, m), 8.43 (1H, m), 8.54 (1H, d, J = 5.9 Hz), 8.78 (1H, d, J = 8.5 Hz), 10.50 (1H, br.s) | A 51 |
| 9 | 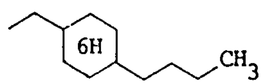 | H | HCl salt colorless prisms 136 - 143 | 3270, 2920, 2600, 1745, 1630, 1550, 1450, 1300, 1185, 1000, 760 (KBr) | 1.75 - 1.80 (19H, m), 4.04 (2H, m), 6.64 (1H, s), 7.70 (1H, br.s), 7.90 (1H, m), 8.46 (1H, m), 8.60 (1H, d, J = 5.3 Hz), 8.74 (1H, d, J = 8.6 Hz), 10.00 (1H, br.s) | B 58 |

Table 2 (Continued)

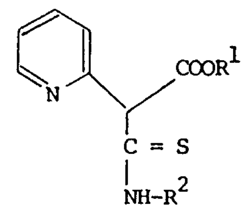

| No. | $R^1$ | $R^2$ | mp°C | IR ν $cm^{-1}$ | NMR($CDCl_3$)σ: | method & yield (%) |
|---|---|---|---|---|---|---|
| 10 | 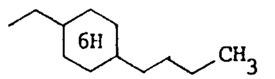 | $CH_3$ | HCl salt colorless prisms 116 - 121 | 3160, 2920, 2600, 1740, 1550, 1470, 1390, 1210, 740 (KBr) | 0.79 - 1.84 (19H, m), 3.13 (3H, d, J = 4.6 Hz), 4.02 (2H, m), 6.63 (1H, s), 7.89 (1H, m), 8.45 (1H, m), 8.58 (1H, d, J = 5.3 Hz), 8.78 (1H, d, J = 8.6 Hz), 10.53 (1H, br.s) | A 74 |
| 11 | 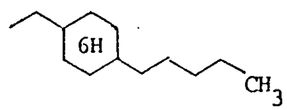 | H | HCl salt colorless prisms 144 - 148 | 3270, 2930, 2600, 1740, 1630, 1450, 1300, 1190, 760 (KBr) | 0.82 - 1.85 (21H, m), 4.03 (2H, m), 6.65 (1H, s), 7.63 (1H, br.s), 7.90 (1H, m), 8.45 (1H, t.d, J = 7.9 Hz), J = 1.3 Hz), 8.58 (1H, d, J = 5.9 Hz), 8.74 (1H, d, J = 7.9 Hz), 10.01 (1H, br.s) | B 70 |
| 12 | 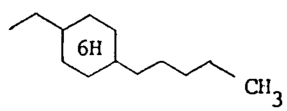 | $CH_3$ | HCl salt colorless prisms 121 - 122 | 3150, 2920, 2600, 1740, 1550, 1470, 1390, 1210, 740 (KBr) | 0.85 - 1.85 (21H, m), 3.13 (3H, d, J = 5.3 Hz), 4.02 (2H, m), 6.64 (1H, s), 7.87 (1H, m), 8.43 (1H, m), 8.53 (1H, d, J = 5.3 Hz), 8.78 (1H, d, J = 8.6 Hz), 10.50 (1H, br.s) | A 91 |

Table 2 (Continued)

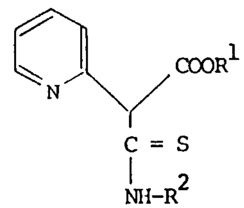

| No. | $R^1$ | $R^2$ | mp°C | IR ν $cm^{-1}$ | NMR($CDCl_3$)σ: | method & yield (%) |
|---|---|---|---|---|---|---|
| 13 | 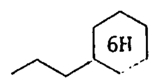 | H | HCl salt colorless prisms 127 - 138 | 3250, 2920, 2600, 1750, 1620, 1440, 1290, 1170, 980, 745 (KBr) | 0.86 - 1.73 (13H, m), 4.15 - 4.39 (2H, m), 6.63 (1H, s), 7.70 (1H, br.s), 7.90 (1H, t, J = 6.6 Hz), 8.46 (1H, t, J = 7.9 Hz), 8.60 (1H, d, J = 5.3 Hz), 8.74 (1H, d, J = 7.9 Hz), 10.00 (1H, br.s) | B 63 |
| 14 | 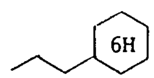 | $CH_3$ | HCl salt colorless prisms 78 - 82 | 3400, 3160, 2930, 1740, 1470, 1390, 1260, 1180, 750 (KBr) | 0.85 - 1.80 (13H, m), 3.13 (3H, d, J = 4.6 Hz), 4.25 (2H, m), 6.63 (1H, s), 7.85 (1H, m), 8.42 (1H, t.d, J = 7.9 Hz, J = 2.0 Hz), 8.52 (1H, d, J = 5.9 Hz), 8.78 (1H, d, J = 7.9 Hz), 10.50 (1H, br.s) | A 70 |
| 15 | 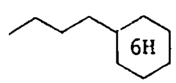 | H | HCl salt colorless prisms 99 - 105 | 3260, 2920, 2840, 2600, 1740, 1630, 1440, 1180, 990, 740 (KBr) | 0.83 - 1.72 (15H, m), 4.21 (2H, m), 6.65 (1H, s), 7.62 (1H, br.s), 7.90 (1H, br.s), 8.46 (1H, m), 8.58 (1H, br.s), 8.75 (1H, d, J = 7.9 Hz), 10.01 (1H, br.s) | B 36 |

Table 2 (Continued)

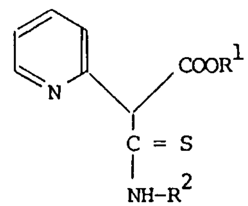

| No. | $R^1$ | $R^2$ | mp°C | IR ν $cm^{-1}$ | NMR($CDCl_3$)σ: | method & yield (%) |
|---|---|---|---|---|---|---|
| 16 | 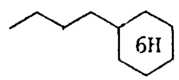 | $CH_3$ | HCl salt colorless prisms 77 - 83 | 3150, 2920, 2600, 1740, 1550, 1470, 1380, 1210, 1170, 990, 745 (KBr) | 0.84 - 1.71 (15H, m), 3.13 (3H, d, J = 4.6 Hz), 4.20 (2H, m), 6.63 (1H, s), 7.87 (1H, m), 8.43 (1H, m), 8.53 (1H, d, J = 5.3 Hz), 8.78 (1H, d, J = 7.9 Hz), 10.51 (1H, br.s) | A 87 |
| 17 | 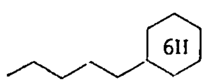 | H | HCl salt colorless prisms 135 - 137 | 3300, 2920, 2600, 1745, 1630, 1450, 1300, 1230, 1170, 1000, 750 (KBr) | 0.80 - 1.77 (17H, m), 4.23 (2H, m), 6.65 (1H, s), 7.60 (1H, br.s), 7.90 (1H, t, J = 7.3 Hz), 8.46 (1H, m), 8.57 (1H, d, J = 5.3 Hz), 8.75 (1H, d, J = 8.6 Hz), 10.02 (1H, br.s) | B 71 |
| 18 | 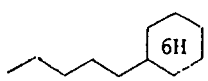 | $CH_3$ | HCl salt colorless prisms 105 - 108 | 3300, 3150, 2920, 2600, 1740, 1550, 1470, 1385, 1210, 745 (KBr) | 0.80 - 1.76 (17H, m), 3.13 (3H, d, J = 4.6 Hz), 4.21 (2H, m), 6.63 (1H, s), 7.87 (1H, m), 8.43 (1H, m), 8.54 (1H, d, J = 5.3 Hz), 8.78 (1H, d, J = 7.9 Hz), 10.52 (1H, br.s) | A 70 |

Table 2 (Continued)

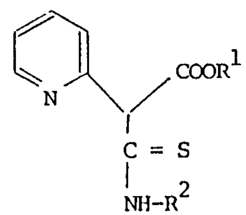

| No. | $R^1$ | $R^2$ | mp°C | IR ν $cm^{-1}$ | NMR ($CDCl_3$) σ: | method & yield (%) |
|---|---|---|---|---|---|---|
| 19 | 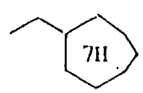 | H | colorless prisms HCl salt 118 - 122 | 3300, 2930, 2600, 1730, 1620, 1460, 1225, 1180, 1005, 760 (KBr) | 1.10 - 2.00 (13H, m), 4.05 (2H, m), 6.63 (1H, s), 7.60 (1H, br.s), 7.91 (1H, m), 8.43 (1H, m), 8.56 (1H, d, J = 4.7 Hz), 8.72 (7.9 Hz), 10.01 (1H, br.s) | B 52 |
| 20 | 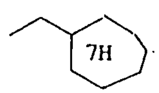 | $CH_3$ | colorless prisms 55 - 56 | 3160, 2920, 1750, 1565, 1480, 1385, 1290, 1165, 745 | 1.05 - 1.78 (12H, m), 3.23 (3H, d, J = 4.6 Hz), 3.90 (2H, m), 5.40 (1H, s), 7.28 (1H, m), 7.44 (1H, d, J = 7.9 Hz), 7.72 (1H, m) 8.55 (1H, d, J = 4.6 Hz), 10.23 (1H, br.s) | A 83 |

| Evaluation Example | |
|---|---|
| Formulated components | Parts by weight |
| Compound of Example<br>Lactose<br>Crystalline cellulose<br>Corn starch | 95<br>25<br>10<br>100 |

The above components were formulated into preparations according to a conventional method.

For the pharmacological examination of the compounds embodying the invention, the following tests were carried out to evaluate the activities of the compounds.

1. Action on hydrochloric acid plus ethanol induced ulcer (protective effect for gastric mucosa)

Sprague-Dawley strain male rats weighing 200 - 240 g were used after starving for 24 hours. To each rat, a 60% ethanolic solution containing 150 mM of hydrochloric acid was administered orally at a volume of 0.5 ml/100 g-body weight, and the stomach was removed under ether anesthesia 1 hour later. Into the stomach, 10 ml of a 2% formalin solution was injected, and the stomach was then immersed in a 2% formalin solution for 15 minutes to fix the inner and outer wall of the stomach. The stomach was cut open along the greater curvature, the length of the damage generated at the glandular portion of the stomach was measured, and the sum of the lengths of damaged gastric mucosa per rat was defined as the lesion index (mm). This was compared with a control group to calculate the percentage of inhibition according to the formula shown below, and then the percentage of inhibition versus dose (mg/kg) was plotted on a semi-logarithmic graph to determine the $ED_{50}$ value. Each test drug was suspended in physiological saline with a few drops of Tween 80® and administered orally 30 minutes before administration of the hydrochloric acid ethanolic solution.

$$\text{Inhibition (\%)} = \frac{\text{Average lesion index of control group} - \text{Average lesion index of group administered with test compound}}{\text{Average lesion index of control group}} \times 100$$

The results of antisecretory effects of gastric acid and protective effects of the gastric mucosa against HCl-EtOH are as shown in Table 3.

2. Ghosh & Schild rats (methods) (inhibitory effect for gastric acid secretion

Male Sprague-Dawley rats (200 - 250 g) were deprived of food but allowed free access to water for 24 hrs. The animals were used under urethane anesthesia (1.25 g/kg, i.p.). The stomach was exposed through a midline incision and a polyethylene cannula was positioned in the forestomach. Another cannula was inserted into the stomach through the duodenum and held in place by a ligature around the pylorus, care being taken not to include blood vessels or bile and pancreatic ducts within the ligature. The stomach was perfused with physiological saline solution at a flow rate of 1 ml/min using a peristaltic pump. Both entry and exit tubes, which were connected to the cannula attached to the forestomach and to the pylorus, were positioned in a reservoir in which a pH glass electrode was placed. The titration was performed at luminal pH 7.0 using a pH-stat method (comtite-8, Hiranuma) and by adding 0.1N NaOH to the reservoir under 100% $O_2$ atmosphere. The acid output was continuously monitored on a recorder. Histamine•2HCl (5 mg/kg/h) was continuously perfused into the tail vein at the rate of 2.2 ml/h. After stabilization of the stimulated acid secretion (90 mm - 2 hr), test samples suspended in 0.5% CMC or Tween® 80 - saline were given intra-duodenally. The acid output was expressed as µEq/30 mm before and 90 - 120 mm after administration of the drug (30 mg/kg i.d.) during the maximally stimulated acid secretion, and the drug suppression of acid secretion was taken as a percentage of mean acid output before administration of the drug.

The data was analyzed with the so-called paired student t-test.

3. Anti stress ulcer

Sprague-Dawley-strain male rats weighing about 240 - 260 g were starved for 24 hours, and 30 minutes after oral administration of the test drug, each rat was placed in a stress cage and stress was loaded by immersing each rat into a water tank maintained at 23°C to the xiphisternum of the chest. Five hours later, the stomach was removed under ether anesthesia, the same treatment as in the case of aspirin ulcer was applied, and the sum of the lengths (mm) of the mucosa damage generated at the glandular portion of the stomach was defined as the lesion index. The $ED_{50}$ value was calculated as described before.

4. Toxicity

To assess toxicity, the test compounds were orally administered in the form of a 0.5% CMC suspension to 5 or 6 weeks old male ddY mice (i.e., 5 mice in each group) to determine the MLD (i.e., minimum lethal dose) thereof.

The results of antiulcer effects on various ulcer models and toxicity are shown in Table 3.

5. Stability test

A 10 mg amount of each compound was dissolved in 100 ml of 0.1 N aqueous hydrochloric acid and was stirred at room temperature for 24 hours. An aqueous sodium hydrogen carbonate solution was added to the resultant solution and the mixture was extracted with chloroform. After washing the extract with water, it was dried over anhydrous magnesium sulfate and the solvent distilled off. The residue was tested by a thin layer chromatograph and by an NMR spectrum to determine whether or not decomposed products were present. Decomposed products were not detected in the compounds embodying the present invention and it was confirmed that the present compounds were stable.

Table 3

| No. | $R^1$ | $R^2$ | Action on HCl-ethanol ulcer $ED_{50}$ mg/kg p.o. | Ghosh & Schild rats 30 mg/kg i.d. inhibition % | Antistress ulcer inhibition %(mg/kg) or ED50(mg/kg) p.o. | Toxicity MLD mg/kg p.o. |
|---|---|---|---|---|---|---|
| 1 | 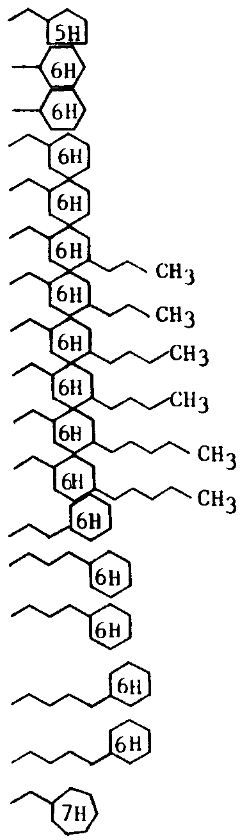 | H | 0.2 | 47 | 67 %(10 mg/kg) | 2197 |
| 3 | | H | 3.7 | 43 | 56 %(10 mg/kg) | 2197 |
| 4 | | $CH_3$ | 4.1 | 5 | -- | >1300 |
| 5 | | H | 5.8 | 50 | ED50= ca.3 | 2856 |
| 6 | | $CH_3$ | 3 - 10 | 44 | 92 %(30 mg/kg) | |
| 7 | | H | 3.5 | 45 | ED50= 4.0 | >2197 |
| 8 | | $CH_3$ | 2.8 | 64 | -- | >1300 |
| 9 | | H | 1.0 | 50 | ED50= 4.0 | >1960 |
| 10 | | $CH_3$ | 2.9 | 54 | -- | 1300 |
| 11 | | H | 1.0 | 45 | 71 %(10 mg/kg) | >2197 |
| 12 | | $CH_3$ | 1.7 | 25 | -- | >1300 |
| 13 | | H | 5.0 | 48 | ED50= 5.3 | >2197 |
| 15 | | H | 2.7 | 58 | ED50= 7.1 | >2197 |
| 16 | | $CH_3$ | 2.8 | 54 | 97 %(30 mg/kg) | 1300 |
| 17 | | H | 3.4 | 37 | ED50= 2.6 | >2197 |
| 18 | | $CH_3$ | 2.2 | 45 | 90 %(30 mg/kg) | 1690 |
| 19 | | H | 1.9 | 63 | 41 %(10 mg/kg) | >2197 |
| Reference | $-C_2H_5$ | H | 11.0 | - | - | - |

As is clear from the results shown in Table 3, the 2-pyridylacetic acids having an $R^1$ of an unsubstituted or substituted cycloalkyl group in the general formula (I) exhibit excellent antiulcer activities, especially superior to those having lower alkyl groups in $R^1$ of the formula (I).

## Claims

1. A compound which is: a 2-pyridylacetic acid derivative having the formula (I)

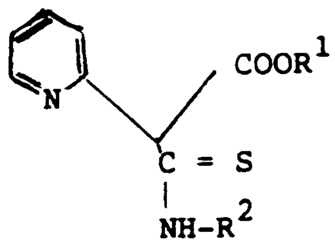

(I)

wherein $R^1$ represents $-(CH_2)_m$-cycloalkyl group having $C_5$ - $C_8$ cycloalkyl, which may be substituted with at least one alkyl group having 1 to 6 carbon atoms, m represents zero or an integer of 1 to 4, and $R^2$ represents hydrogen or a linear $C_{1-10}$ alkyl; or a pharmacologically acceptable acid addition salt thereof.

2. A compound according to claim 1, for pharmaceutical use.

3. A compound according to claim 1, for use in the treatment of peptic ulcer.

4. A therapeutic agent for treating peptic ulcer, comprising a therapeutically effective amount of a compound according to claim 1.

5. A process comprising the preparation of a compound according to claim 1,

6. A process according to claim 5, comprising
(i) treating a compound of the formula (II)

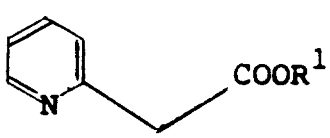

(II)

with a base at a temperature of 0 °C or lower, and
(ii) reacting the product of step (i) with an isothiocyanate of the formula (V)

$S=C=N-R^3$ (V)

wherein $R^3$ is an alkyl group.

7. A process according to claim 5 comprising reacting a compound of the formula (III)

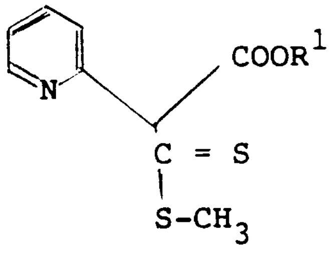

(III)

with ammonia or with an amine of the formula (IV)

$R^2$-$NH_2$ (IV)

**8.** A process according to claim 7 wherein a compound of the formula (II)

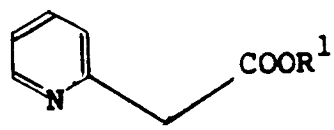

(II)

is reacted with carbon disulphide at a temperature of from - 78°C to 0°C, and with methyl iodide, to make the compound of formula (III).

**9.** A process according to any one of claims 5 to 8, further comprising converting the derivative of formula (I) to a pharmacologically acceptable acid addition salt thereof.

**10.** Use of a compound according to claim 1 in the manufacture of a medicament for the treatment of peptic ulcer.

**11.** A compound according to any one of claims 1 to 3, therapeutic agent according to claim 4, process according to any one of claims 5 to 9, or use according to claim 10, in which the 2-pyridylacetic acid derivative is any one of:
Cyclopentylmethyl 2-(2-pyridyl)-2-thiocarbamoylacetate hydrochloride,
Cyclopentylmethyl 2-methylthiocarbamoyl-2-(2-pyridyl)acetate,
Cyclohexyl 2-(2-pyridyl)-2-thiocarbamoylacetate hydrochloride
Cyclohexyl 2-methylthiocarbamoyl-2-(2-pyridyl)acetate,
Cyclohexylmethyl 2-(2-pyridyl)-2-thicarbamoylacetate hydrochloride,
Cyclohexylmethyl 2-methylthiocarbamoyl-2-(2-pyridyl)acetate hydrochloride,
(trans-4-n-Propylcyclohexyl)methyl 2-(2-pyridyl)-2-thiocarbamoylacetate hydrochloride,
(trans-4-n-Propylcyclohexyl)methyl 2-methyl-thiocarbamoyl-2-(2-pyridyl)acetate hydrochloride,
(trans-4-n-Butylcyclohexyl)methyl 2-(2-pyridyl)-2-thiocarbamoylacetate hydrochloride,
(trans-4-n-Butylcyclohexyl)methyl 2-methylthiocarbamoyl-2-(2-pyridyl)acetate hydrochloride,
(trans-4-n-Pentylcyclohexyl)methyl 2-(2-pyridyl)-2-thiocarbamoylacetate hydrochloride,
(trans-4-n-Pentylcyclohexyl)methyl 2-methylthiocarbamoyl-2-(2-pyridyl)acetate hydrochloride,
Cyclohexylethyl 2-(2-pyridyl)thiocarbamoylacetate hydrochloride,
Cyclohexylethyl 2-methylthiocarbamoyl-2-(2-pyridyl)acetate hydrochloride,
Cyclohexylpropyl 2-(2-pyridyl)-2-thiocarbamoylacetate hydrochloride,
Cyclohexylpropyl 2-methylthiocarbamoyl-2-(2-pyridyl)acetate hydrochloride,
Cyclohexylbutyl 2-(2-pyridyl)-2-thiocarbamoylacetate hydrochloride,
Cyclohexylbutyl 2-methylthiocarbamoyl-2-(2-pyridyl)acetate hydrochloride,
Cycloheptylmethyl 2-(2-pyridyl)-2-thiocarbamoylacetate hydrochloride, and
Cycloheptylmethyl 2-methylthiocarbamoyl-2-(2-pyridyl)acetate.

**Patentansprüche**

**1.** Verbindung, die ein 2-Pyridylessigsäurederivat der Formel (I)

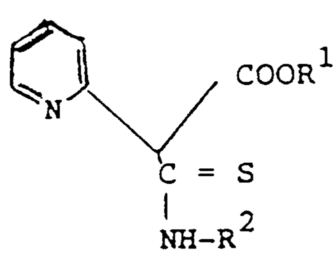

(I)

in der $R^1$ eine $-(CH_2)_m$-Cycloalkylgruppe mit $C_5$-$C_8$-Cycloalkyl darstellt, die mit zumindest einer Alkylgruppe mit 1 bis 6 Kohlenstoffatomen substituiert sein kann, m 0 oder eine ganze Zahl von 1 bis 4 ist und $R^2$ Wasserstoff oder ein lineares $C_1$-$C_{10}$-Alkyl darstellt, oder deren pharmakologisch annehmbares Säureadditionssalz ist.

**2.** Verbindung nach Anspruch 1 zur pharmazeutischen Verwendung.

**3.** Verbindung nach Anspruch 1 zur Verwendung in der Behandlung eines Magengeschwürs.

**4.** Therapeutisches Mittel zur Behandlung eines Magengeschwürs, enthaltend eine therapeutisch wirksame Menge einer Verbindung nach Anspruch 1.

**5.** Verfahren enthaltend die Herstellung einer Verbindung nach Anspruch 1.

**6.** Verfahren nach Anspruch 5,
**dadurch gekennzeichnet,** daß
(i) eine Verbindung der Formel (II)

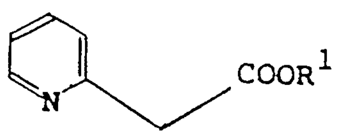

(II)

mit einer Base bei einer Temperatur von 0 °C oder darunter behandelt und
(ii) das Produkt der Stufe (i) mit einem Isothiocyanat der Formel (V)

$S=C=N-R^3$ (V) ,

in der $R^3$ eine Alkylgruppe ist, umgesetzt wird.

**7.** Verfahren nach Anspruch 5,
**dadurch gekennzeichnet,** daß
eine Verbindung der Formel (III)

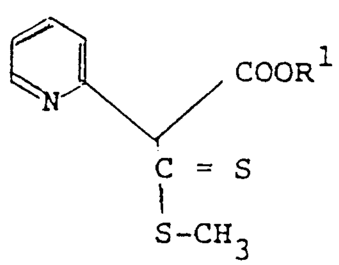

(III)

mit Ammoniak oder mit einem Amin der Formel (IV)

$R^2$-$NH_2$ (IV)

umgesetzt wird.

**8.** Verfahren nach Anspruch 7,
**dadurch gekennzeichnet,** daß
eine Verbindung der Formel (II)

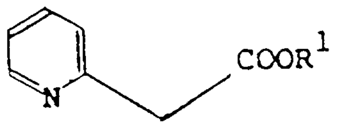

(II)

mit Kohlenstoffdisulfid bei einer Temperatur von -78°C bis 0°C und mit Methyliodid umgesetzt wird, um die Verbindung der Formel (III) zu bilden.

**9.** Verfahren nach einem der Ansprüche 5 bis 8,
**dadurch gekennzeichnet,** daß
das Derivat der Formel (I) zu derem pharmakologisch annehmbaren Säureadditionssalz umgewandelt wird.

**10.** Verwendung einer Verbindung nach Anspruch 1 zur Herstellung eines Medikaments zur Behandlung eines Magengeschwürs.

**11.** Verbindung nach einem der Ansprüche 1 bis 3, therapeutisches Mittel nach Anspruch 4, Verfahren nach einem der Ansprüche 5 bis 9 oder Verwendung nach Anspruch 10,
**dadurch gekennzeichnet, daß**
das 2-Pyridylessigsäurederivat eines der nachfolgenden ist:
Cyclopentylmethyl-2-(2-pyridyl)2-thiocarbamoylacetat-hydrochlorid,
Cyclopentylmethyl-2-methylthiocarbamoyl-2-(2-pyridyl)acetat,
Cyclohexyl-2-(2-pyridyl)-2-thiocarbamoylacetat-hydrochlorid,
Cyclohexyl-2-methylthiocarbamoyl-2-(2-pyridyl)acetat,
Cyclohexylmethyl-2-(2-pyridyl)-2-thiocarbamoylacetat-hydrochlorid,
Cyclohexylmethyl-2-methylthiocarbamoyl-2-(2-pyridyl)acetat-hydrochlorid,
(trans-4-n-Propylcyclohexyl)methyl-2-(2-pyridyl)-2-thiocarbamoylacetat-hydrochlorid,
(trans-4-n-Propylcyclohexyl)methyl-2-methylthiocarbamoyl-2-(2-pyridyl)acetat-hydrochlorid,
(trans-4-n-Butylcyclohexyl)methyl-2-(2-pyridyl)-2-thiocarbamoylacetat-hydrochlorid,
(trans-4-n-Butylcyclohexyl)methyl-2-methylthiocarbamoyl-2-(2-pyridyl)acetat-hydrochlorid,
(trans-4-n-Pentylcyclohexyl)methyl-2-(2-pyridyl)-2-thiocarbamoylacetat-hydrochlorid,
(trans-4-n-Pentylcyclohexyl)methyl-2-methylthiocarbamoyl-2-(2-pyridyl)acetat-hydrochlorid,
Cyclohexylethyl-2-(2-pyridyl)-thiocarbamoylacetat-hydrochlorid,
Cyclohexylethyl-2-methylthiocarbamoyl-2-(2-pyridyl)acetat-hydrochlorid,
Cyclohexylpropyl-2-(2-pyridyl)-2-thiocarbamoylacetat-hydrochlorid,
Cyclohexylpropyl-2-methylthiocarbamoyl-2-(2-pyridyl)acetat-hydrochlorid,
Cyclohexylbutyl-2-(2-pyridyl)-2-thiocarbamoylacetat-hydrochlorid,
Cyclohexylbutyl-2-methylthiocarbamoyl-2-(2-pyridyl)acetat-hydrochlorid,
Cycloheptylmethyl-2-(2-pyridyl)-2-thiocarbamoylacetat-hydrochlorid und
Cycloheptylmethyl-2-methylthiocarbamoyl-2-(2-pyridyl)acetat.

## Revendications

**1.** Un composé qui consistant en un dérivé d'acide 2-pyridylacétique de formule (I):

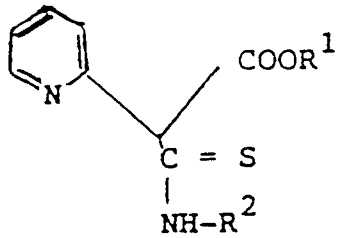

(I)

dans laquelle:

$R^1$ représente un groupe cycloalkyle $-(CH_2)_m$ comportant un groupe cycloalkyle en $C_5$ à $C_8$ qui

peut être substitué par au moins un groupe alkyle ayant 1 à 6 atomes de carbone;
m est égal à zéro ou est un entier de 1 à 4; et
$R^2$ est un atome d'hydrogène ou un groupe alkyle linéaire en $C_1$-$C_{10}$,
ou un sel d'addition d'acide pharmacologiquement acceptable en dérivant.

**2.** Un composé selon la revendication 1 pour une utilisation pharmaceutique.

**3.** Un composé selon la revendication 1 pour une utilisation dans le traitement de l'ulcère gastroduodénal ou peptique.

**4.** Un agent thérapeutique pour le traitement de l'ulcère duodénal comprenant une quantité efficace du point de vue thérapeutique d'un des composés selon la revendication 1.

**5.** Un procédé comprenant la préparation du composé selon la revendication 1.

**6.** Un procédé selon la revendication 5, comprenant:
(i) le traitement d'un composé de formule (II):

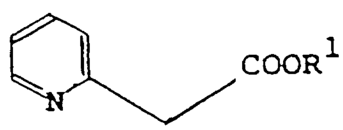

(II)

par une base à une température de 0 °C ou inférieure; et
(ii) la réaction du produit de l'étape (i) avec un isothiocyanate de formule (V):

$S = C = N - R^3$ (V)

dans laquelle $R^3$ est un radical alkyle.

**7.** Un procédé suivant la revendication 5 comprenant la réaction d'un composé de formule (III):

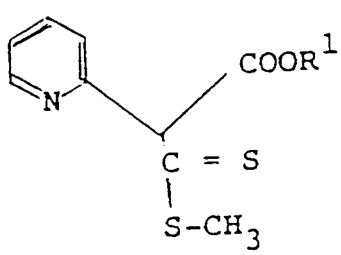

(III)

avec de l'ammoniac ou avec une amine de formule (IV):

$R^2 - NH_2$ (IV)

**8.** Un procédé selon la revendication 7 dans lequel le composé de formule (II):

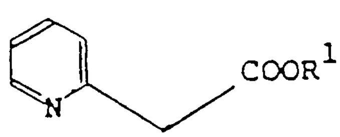

(II)

réagit avec un bisulfure de carbone à une température de -78 à 0 °C, et avec de l'iodure de méthyle, pour fournir un composé de formule (III).

**9.** Un procédé selon l'une quelconque des revendications 5 à 8 comprenant de plus la conversion du dérivé de formule (I) en un de ses sels d'addition d'acide pharmacologiquement acceptable en dérivant.

**10.** L'utilisation d'un composé selon la revendication 1 dans la fabrication d'un médicament pour le traitement de l'ulcère gastroduodénal.

**11.** Un composé selon l'une quelconque des revendications 1 à 3, un agent thérapeutique selon la revendication 4, un procédé selon l'une quelconque des revendications 5 à 9, ou l'utilisation selon la revendication 10 dans lesquelles le dérivé d'acide 2-pyridylacétique est l'un quelconque de ces composés suivants:

. Chlorhydrate de cyclopentylméthyl-2-(2-pyridyl)-2-thiocarbamoylacétate
. Cyclopentylméthyl-2-méthylthiocarbamoyl-2-(2-pyridyl)-acétate
. Chlorhydrate de cyclohexyl-2-(2-pyridyl)-2-thiocarbamoylacétate
. Cyclohexyl-2-méthylthiocarbamoyl-2-(2-pyridyl)-acétate
. Chlorhydrate de cyclohexylméthyl-2-(2-pyridyl)-2-thiocarbamoylacétate
. Chlorhydrate de cyclohexylméthyl-2-méthylthiocarbamoyl-2-(2-pyridyl)acétate
. Chlorhydrate de (trans-4-n-propylcyclohexyl)-méthyl-2-(2-pyridyl)-2-thiocarbamoylacétate
. Chlorhydrate de (trans-4-n-propylcyclohexyl)-méthyl-2-méthyl-thiocarbamoyl-2-(2-pyridyl)acétate
. Chlorhydrate de (trans-4-n-butylcyclohexyl)-méthyl-2-(2-pyridyl)-2-thiocarbamoylacétate
. Chlorhydrate de (trans-4-n-butylcyclohexyl)-méthyl-2-méthylthiocarbamoyl-2-(2-pyridyl)acétate
. Chlorhydrate de (trans-4-n-pentylcyclohexyl)méthyl-2-(2-pyridyl)-2-thiocarbamoylacétate
. Chlorhydrate de (trans-4-n-pentylcyclohexyl)méthyl-2-méthylthiocarbamoyl-2-(2-pyridyl)acétate
. Chlorhydrate de cyclohexyléthyl-2-(2-pyridyl)-thiocarbamoylacétate
. Chlorhydrate de cyclohexyléthyl-2-méthylthio-carbamoyl-2-(2-pyridyl)acétate
. Chlorhydrate de cyclohexylpropyl-2-(2-pyridyl)-2-thiocarbamoylacétate
. Chlorhydrate de cyclohexylpropyl-2-méthylthio-carbamoyl-2-(2-pyridyl)acétate
. Chlorhydrate de cyclohexylbutyl-2-(2-pyridyl)-2-thiocarbamoylacétate
. Chlorhydrate de cyclohexylbutyl-2-méthylthio-carbamoyl-2-(2-pyridyl)acétate
. Chlorhydrate de cycloheptylméthyl-2-(2-pyridyl)-2-thiocarbamoylacétate, et
. Cycloheptylméthyl-2-méthylthiocarbamoyl-2-(2-pyridyl)-acétate.